# EUROPEAN PATENT APPLICATION

(11) **EP 4 309 593 A1**
(43) Date of publication of application: **24.01.2024**
(21) Application number: 23185807.7
(22) Date of filing: 17.07.2023
(51) Int. Cl.: A61B 17/135

(54) **TIMED ANTI-BLEEDING TOURNIQUET**

(30) Priority: 18.07.2022 TR 202211471
(71) Applicant: Sivas Cumhuriyet Universitesi Rektorlugu, 58140 Sivas Sivas (TR)
(72) Inventor: SARI, Ayse, Sivas (TR); TURKMENOGLU, Bahar, Sivas (TR)
(74) Representative: Berkkam, Ayfer

(57) **Abstract**

The invention relates to a tourniquet that aims to stop bleeding by applying pressure to the blood vessels in the extremities (arms and legs) with a compressed air system for a certain period of time in order to stop serious bleeding in accidents and injuries that pose a life-threatening danger as a result of the blood circulating in the vessels coming out for various reasons.

## Description

### Technical Field

The invention relates to a tourniquet that aims to stop bleeding by applying pressure to the blood vessels in the extremities (arms and legs) with a compressed air system for a certain period of time in order to stop serious bleeding in accidents and injuries that pose a life-threatening danger as a result of the blood circulating in the vessels coming out for various reasons.

### Prior Art

Tourniquet is an application used to stop vascular bleeding in the limb in case of limb rupture in arm and/or leg injuries.

In the known state of the technique, there are studies on stopping the blood flow by wrapping an elastic and soft material over the bleeding area to stop bleeding. Bandages/fabric pieces are wrapped around the arm and foot limbs and knots are tied. Materials such as a piece of wood or a pen are placed in the center of the knot. By rotating this piece of wood, the bandage creates a pressure that squeezes the limb. The rotation of the board continues until the bleeding stops. The time of the tourniquet procedure is written on a piece of paper and hung on the wound. The bandage, which is tightened for blood supply to the organ, is loosened at intervals of 15-20 minutes. In this case, the pressure applied may vary. As a result, it may lead to organ damage or loss.

In the United States patent document numbered US4469099A, which is in the known state of the art, a pneumatic tourniquet comprising an inflatable sleeve, a pressurizing mechanism for pressurizing the sleeve, a pressure relief mechanism for depressurizing the sleeve, a pressure sensing mechanism for sensing the pressure at which the sleeve is pressurized, and a pressure regulator mechanism for selectively activating the pressurizing mechanism is mentioned. It includes an electric air pump as a means of pressure. A hose is used for pressure adjustment. Visual and audible alarm signals are triggered if the sleeve pressure exceeds the sleeve pressurization limit, falls below the pressurization limit, or if the sleeve remains pressurized for a selected time or longer.

In the International patent document numbered WO2017121914A1, which is in the known state of the art, there is mention of a hygienic tourniquet formed by a strap with a fastener at the end and a tooth on the upper face and at least one trigger. The latch is pivotably connected to a buckle in a ratchet mechanism. The distal end of the button is inserted into the strap buckle, which engages the teeth of the latch and prevents the trigger from being released until the trigger is forced. The trigger has a tongue for release by pressing. The trigger includes an additional band that is secured to the trigger and lifts the trigger to release it when pulled. The tourniquet includes support means for identification, personalization and information, such as a label, barcode or chip.

In the United States patent document US7485131B2, which is in the known state of the art, there is a system and method for controlling the pressure within a pressure sleeve of a surgical tourniquet to selectively occlude blood flow in a part of a limb of a patient, wherein a sensor determines when flow through the tourniquet occurs. This is said to allow corrective action to be taken. The tourniquet controller can gradually increase the pressure in the tourniquet or give an alarm signal indicating blood flow if a threshold is exceeded by such an increase. It is mentioned that a pump is used as a source of pressure.

Israeli patent document IL179769A, which is in the known state of the art, discloses an outer sleeve, an inner strap slidably interlocking with the outer sleeve, an upper panel connected to a lower panel, and a portion of the upper panel comprising a hook and loop fastener. There is also a single component hook and loop fastener.

However, in the systems included in the exemplary patent documents, it was necessary to develop the inventive timed anti-bleeding tourniquet, which allows a warning to be given to loosen the tourniquet after a certain period of time during the tightening phase of the tourniquet.

### Purpose of the Invention

The object of the present invention is to provide a timed anti-bleeding tourniquet that provides a reminder to loosen the limb at certain intervals during the tourniquet application phase.

### Detailed Description of the Invention

A timed anti-bleeding tourniquet for achieving the objects of the present invention is shown in the accompanying figures.

These figures;

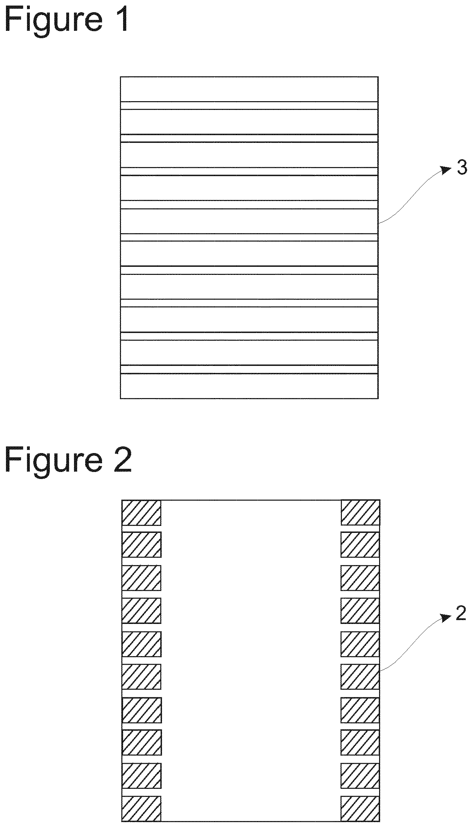

**Figure 1****:** Appearance of the sleeve retaining iron in the inventive tourniquet.
**Figure 2****:** The appearance of the sleeve retaining iron connection in the inventive tourniquet.

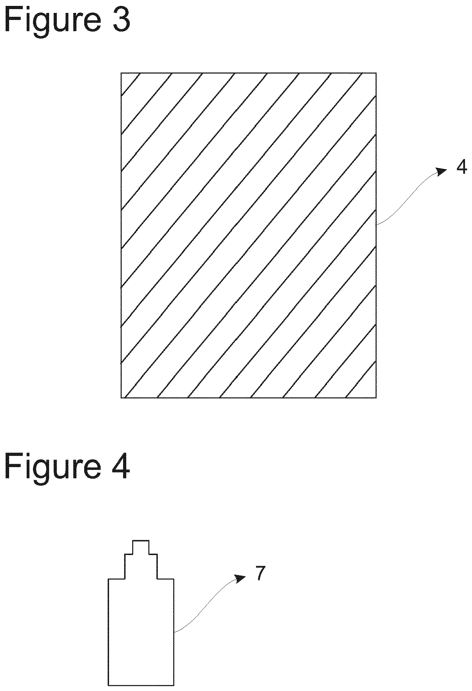

**Figure 3****:** The appearance of the sleeve retaining hook-and-loop system in the inventive tourniquet.
**Figure 4****:** View of the bulb cable entrance in the inventive tourniquet.

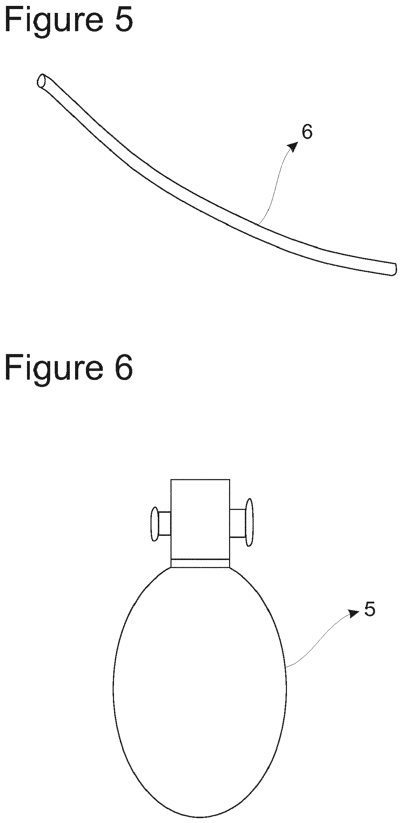

**Figure 5****:** View of the spare inflation bulb hose in the inventive tourniquet.
**Figure 6****:** View of the bulb on the inventive tourniquet.
**Figure 7****:** Schematic front view of the inventive tourniquet.

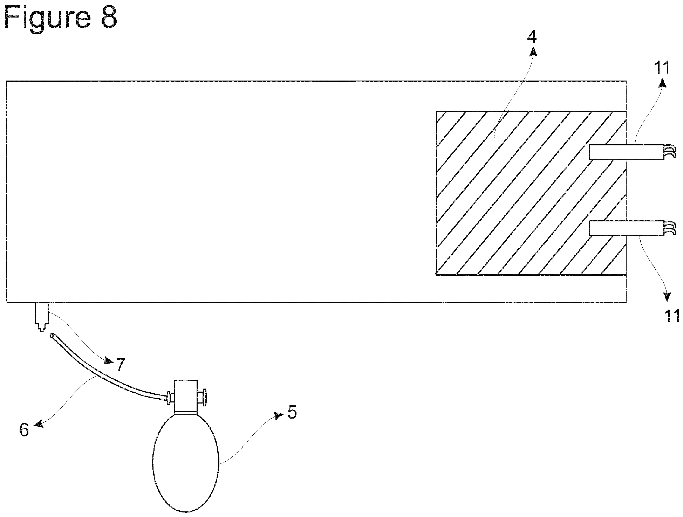

**Figure 8****:** Schematic view of the inventive tourniquet from the back.

The parts in the figures are numbered one by one and the equivalents of these numbers are given below.
1. Main sleeve
2. Sleeve retaining iron
3. Sleeve retaining iron connection
4. Hook-and-loop connection
5. Bulb
6. Spare inflation bulb hose
7. Bulb cable entry
8. Alarm system
9. Inflation button
10. Transparent area
11. Hooks
12. Air release button

The invention relates to a timed anti-bleeding tourniquet comprising
- a main sleeve consisting of a rectangular cloth structure to be wrapped around the injured area for tourniquet (1),
- a sleeve retaining iron (3) placed on the sleeve retaining iron connection (2) on the main sleeve (1) and securing the tourniquet,
- hook-and-loop connection (4) at the inner and outer ends of the main sleeve (1), which allows the ends of the main sleeve (1) to be held together,
- a rubber bulb (5) for supplying air to the main sleeve (1),
- a spare inflation bulb hose (6) which will ensure air passage between the main sleeve (1) and the bulb (5),
- a bulb cable inlet (7) located on the main sleeve (1) and allowing manual air inlet to the tourniquet,
- a minute-set alarm system located on the main sleeve (1) and used for time control during tourniquet application (8),
- an inflation button (9) located on the main sleeve (1), which allows the tourniquet to inflate and the alarm system (8) to operate,
- a transparent area (10) on the main sleeve (1) with a large red letter T (indicating that a tourniquet has been applied) and a small red pen, allowing the time of tourniquet application to be written on it,
- hooks (11) located on the main sleeve (1) and enabling the main sleeve (1) to be connected to the sleeve holder iron (2),
- an air release button (12) located on the main sleeve (1), which allows the air in the main sleeve (1) to escape.

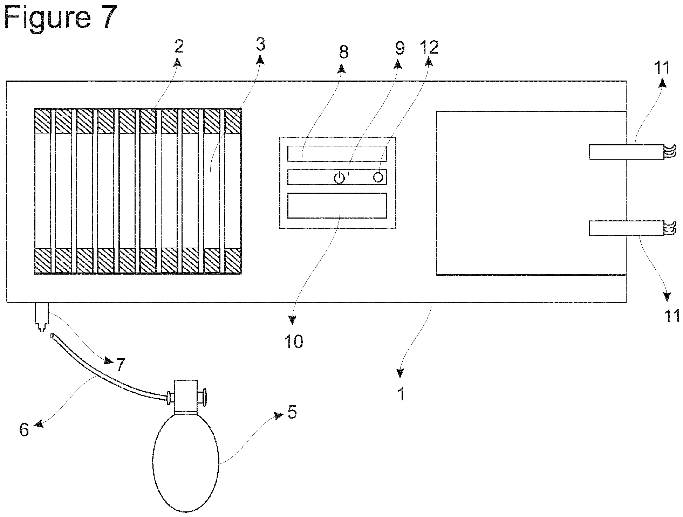

The front view of the inventive tourniquet is shown in Figure 7. The tourniquet is wrapped around the intact area over the bleeding area on the arm or leg, encircling the limb. The hooks (11) are attached to the sleeve holder iron connection (3). At the same time, the ends of the main sleeve (1) are held together with hook and loop fasteners (4). The main sleeve (1) is inflated by pressing the inflation button (9). The inflation process is continued until the bleeding stops. When the inflation button (9) is pressed a second time, inflation is stopped. The inflation button (9) and the alarm system (8) work in integration. The alarm system (8) starts the 15 minute countdown and when the 15 minutes are over, the alarm system (8) gives an audible warning. The application time is written on the transparent area (10) to draw attention to the tourniquet application. The red colored letter T in the transparent area (10) is pasted on the forehead of the injured/victim, which is the most conspicuous area. If there is a problem with sticking, the first assistant writes T with a pen. With the alarm sound, the air deflation button (12) is pressed to deflate the inflated main sleeve (1). At the same time, if there is a problem in the inflation of the main sleeve (1) due to a mechanical fault in the tourniquet, the inflation is provided manually with the bulb (5) by combining the bulb inlet (6) and the bulb cable (7) as a backup.

The developed timed anti-bleeding tourniquet aims to stop serious bleeding in life-threatening accidents and injuries as a result of the blood circulating in the veins coming out for various reasons. The inventive tourniquet stops bleeding by applying pressure to the blood vessels in the arms and legs for certain periods of time with a compressed air system.

The advantages obtained with the developed device are listed below.
- In case of limb rupture in arm and/or leg injuries, it will ensure that the vascular bleeding occurring in the limb stops.
- During the tourniquet application phase, blood flow to the organ is ensured by warning the limb to be loosened at certain intervals. Thus, organ damage or loss can be prevented.

## Claims

1. The invention relates to a timed anti-bleeding tourniquet **characterized in that** it comprises
- a main sleeve (1) consisting of a rectangular cloth structure to be wrapped around the injured area for tourniquet,
- a sleeve retaining iron (3) placed on a sleeve retaining iron connection (2) on the main sleeve (1) and securing the tourniquet,
- hook-and-loop connection (4) at the inner and outer ends of the main sleeve (1), which allows the ends of the main sleeve (1) to be held together,
- a minute-set alarm system (8) located on the main sleeve (1) and used for time control during tourniquet application.

2. The invention relates to a timed anti-bleeding tourniquet according to claim 1, **characterized in that** it comprises a rubber bulb (5) for supplying air to the main sleeve (1).

3. The invention relates to a timed anti-bleeding tourniquet according to claim 1, **characterized in that** it comprises a spare inflation bulb hose (6) for air passage between the main sleeve (1) and the bulb (5).

4. The invention relates to a timed anti-bleeding tourniquet according to claim 1, **characterized in that** it comprises a bulb cable inlet (7) located on the main sleeve (1) and allowing manual air intake into the tourniquet.

5. The invention relates to a timed anti-bleeding tourniquet according to claim 1, **characterized in that** it comprises an on-off switch (9) located on the main sleeve (1) and allowing the alarm system (8) to be switched on and off.

6. The invention relates to a timed anti-bleeding tourniquet according to claim 1, **characterized in that** it comprises a transparent area (10) on the main sleeve (1) which allows the time of application of the tourniquet to be written on it.

7. The invention relates to a timed anti-bleeding tourniquet according to claim 1, **characterized in that** it comprises hooks (11) located on the main sleeve (1) and enabling the main sleeve (1) to be connected to the sleeve retaining iron (2).

8. The invention relates to a timed anti-bleeding tourniquet according to claim 1, **characterized in that** it comprises an air release button (12) located on the main sleeve (1) and allowing the air contained in the main sleeve (1) to escape.
